# EUROPEAN PATENT SPECIFICATION

(11) **EP 3 687 608 B1**
(45) Date of publication and mention of the grant of the patent: **18.06.2025**
(21) Application number: 18860532.3
(22) Date of filing: 25.09.2018
(51) Int. Cl.: A61M 15/00

(54) **DRY POWDER INHALER WITH MEANS FOR ENHANCING DISPERSION**
TROCKENPULVERINHALATOR MIT MITTELN ZUR VERBESSERUNG DER DISPERSION
INHALATEUR DE POUDRE SÈCHE COMPRENANT DES MOYENS PERMETTANT D'AMÉLIORER LA DISPERSION

(30) Priority: 28.09.2017 SE 1751199
(43) Date of publication of application: 05.08.2020
(73) Proprietor: Iconovo AB, 223 63 Lund (SE)
(72) Inventor: LASTOW, Orest, 247 45 Torna Hällestad (SE); ARVIDSSON, Lars, 247 51 Dalby (SE); JOHNSON, Richard, 21245 Malmö (SE); GUNNARSON, Fredrik, 247 34 Södra Sandby (SE)
(74) Representative: Ström & Gulliksson AB
(86) International application number: PCT/SE2018/050971
(87) International publication number: WO 2019/066702

(56) References cited:
- EP-A1- 0 069 715
- WO-A1-2016/180752
- WO-A1-2016/180753
- GB-A- 2 041 763
- US-A- 4 907 583
- US-A- 5 660 169
- US-A1- 2005 056 281
- US-A1- 2012 247 465
- US-A1- 2016 367 769

## Description

### FIELD OF THE INVENTION

This invention pertains in general to the field of dry powder inhalers. Even more particularly, the invention pertains to a dry powder inhaler comprising a turbulence enhancing protrusion (TEP).

### BACKGROUND OF INVENTION

Inhalers have been widely used in the pharmaceutical field for treatment of respiratory and/or other diseases. Numerous drugs, medications and other substances are inhaled into the lungs using the inhalers for rapid absorption of the drug etc. in the blood stream and for local action in the lung.

Inhaled drugs fall into two main categories, one being in the form of liquids, including suspensions, and the other being powders. The choice of liquids or powders depends on the characteristics of the drugs, medications, etc. to be inhaled.

The most common type of inhaler is the pressurized metered-dose inhaler. In this type of inhaler medication is most commonly stored in solution in a pressurized canister that contains a propellant, although it may also be a suspension. The canister is attached to a plastic, hand-operated actuator. On activation, the metered-dose inhaler releases a fixed dose of medication in aerosol form.

Another kind of inhaler is a nebulizer, which supplies medication as an aerosol created from an aqueous formulation.

The kind referred to herein is yet another type, in the form of a dry powder inhaler. A dry powder inhaler releases a pre-metered, capsuled, dose or a device-metered dose of powdered medication that is inhaled through the inhaler. Inhalers with a device-metered dose of powdered medication are normally inhalers with a medication reservoir containing powdered medication, from which metered doses are withdrawn through the use of different dose metering arrangements, the doses then being inhaled.

Dry powder inhalers need to deliver a particle size that is predominantly below 5 microns, and preferably between 1 micron and 3.3 microns, for maximum effectiveness. However, such small particles are often very cohesive due to high surface energy. Agglomeration may be worsened by moisture and / or when the medication comprises more than one active substance, since the different active substances may have such properties as to form agglomerations with each other or with pharmaceutical carriers etc. Agglomeration of small particles is a problem which results in the active particles leaving the inhaler as large agglomerates.

EP0237507 relates to a device in powder inhalators intended to be used for local administration of drugs to the respiratory tract and lungs of a patient.

As such, there exists a need for an improved dry powder inhaler device in which effective and satisfactory dispersion of the dry powder is obtained and which inhaler efficiently facilitates deaggregation and dispersion.

GB2041763A, EP0069715A1, US2012247465A1, and US4907583A disclose prior art inhalers.

### SUMMARY OF INVENTION

Accordingly, the present invention preferably seeks to mitigate, alleviate or eliminate one or more of the above-identified deficiencies in the art and disadvantages singly or in any combination and solves at least the above mentioned problems by providing a dry powder inhaler as defined in claim 1.
Further advantageous embodiments are disclosed below and in the appended patent claims.

### BRIEF DESCRIPTION OF THE DRAWINGS

These and other aspects, features and advantages of which the invention is capable will be apparent and elucidated from the following description of embodiments of the present invention, reference being made to the accompanying drawings, in which
Fig. 1 is a cross sectional view along a longitudinal axis of an inhaler in the dose administering position according to one embodiment of the present invention;
Fig. 2 is a perspective and cross sectional view of the inhaler in Fig. 1, wherein air inlets are omitted from Fig. 2 for clarity;
Fig. 3 is a cross sectional view along a longitudinal axis of the inhaler in Fig. 1 wherein the air and air/medicament flows through the inhaler are disclosed;
Fig. 4a-c show detailed views of the inhaler according to embodiments;
Fig. 5 is a cross sectional view of details of the inhaler according to an embodiment;
Fig. 6 is a cross sectional view of details of the inhaler in Fig. 1;
Fig. 7 is a cross sectional view along a transverse plane showing details of the inhaler in Fig. 1;
Fig. 8 is another cross sectional view along a longitudinal plane showing details of the inhaler according to an embodiment;
Fig. 9a-b is a perspective view showing a medicament scraper without TEP and;
Fig. 9c-d is a medicament scraper according to one embodiment for use in the inhaler in Fig. 1;
Fig. 10a-e shows bottom views of five exemplary embodiments of the medicament scraper for use in the inhaler in Fig. 1; and
Fig. 11a-b shows side views of embodiments of the medicament scraper for use in the inhaler in Fig. 1.

### DETAILED DESCRIPTION OF EMBODIMENTS

The following description focuses on embodiments of the present invention applicable to a medicament inhaler 100, and in particular to a dry powder drug inhaler with more than one medicament reservoir, such as two medicament reservoirs. However, it will be appreciated that the invention is not limited to this application but may be applied to many other inhalers having an inlet and an outlet, as well as a medicament reservoir.

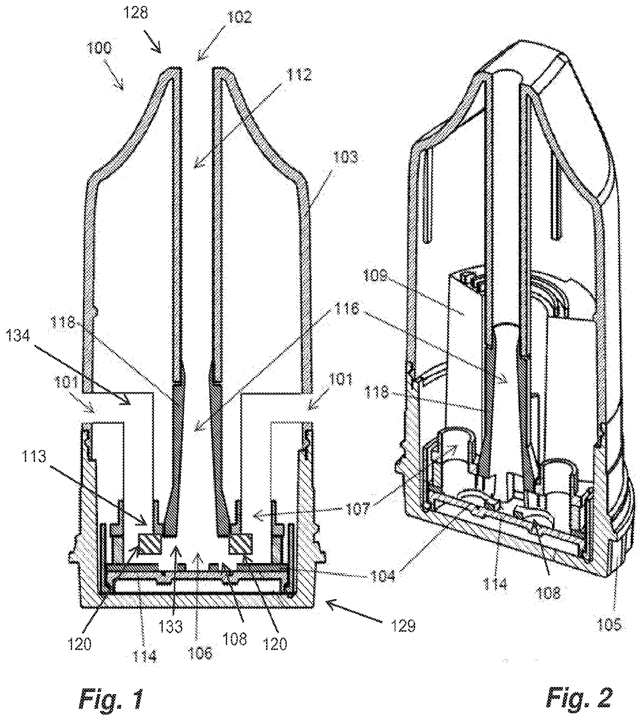

Fig. 1 and 2 illustrate a dry powder drug inhaler 100. The dry powder drug inhaler 100 comprises air inlets 101 and an air outlet 102. The outlet 102 is arranged in a zone of a proximal end 128 of the dry powder drug inhaler 100 while the inlets 101 are arranged at a zone in an opposite distal end 129 of the dry powder drug inhaler 100. The outlet 102 is arranged centrally along the longitudinal axis of the dry powder drug inhaler 100. The inlets 101 may be arranged at the periphery of the dry powder inhaler 100 in a radial position in relation to the longitudinal axis of the dry powder drug inhaler 100, such that the inlets 101 lead inhaled air transversally and radially towards the central portion of the dry powder inhaler 100.

Although not illustrated in Figs. 1 and 2, the inlets 101 may also be positioned with a direction that is parallel to the central axis of the dry powder inhaler 100.

The number of inlets and outlets may be different from what is disclosed in Figs. 1 and 2. The number of inlets may for example be adjusted in accordance with needs and specific inhaler design such that a number of smaller air inlets, for reducing pressure fall over the inhaler, are arranged circumferentially on the dry powder inhaler 100. In a similar manner the number of air outlets may be adjusted in accordance with needs and specific inhaler design.

The different parts of the dry powder inhaler 100 may be manufactured in a suitable material, such as injection moldable plastics, such as thermoplastics.

The dry powder inhaler 100 comprises three major parts in the form of (i) an upper proximal reservoir housing 103 with an inhalation chimney 112, (ii) a dosage mechanism 118 comprising a dose disc 104 having at least one cavity 108, a mixing and deaggregation chamber 106 adjacent to the at least one cavity 108, and a conduit 116 extending distally from the chamber 106, and (iii) a lower distal twister 105 which in some embodiments may comprise a floor disc 114. The reservoir housing 103 and the twister 105 cooperate so as to house the dosage mechanism 118 and the floor disc 114 in between housing 103 and twister 105. The chimney 112 of the reservoir housing 103 cooperates with the conduit 116 of the dosage mechanism 118 such that the dose disc 104 may be rotated between a dose administering position and a dose collecting position when the reservoir housing 103 is rotated. The floor disc 114 is connected to twister 105 so that floor disc 114 only moves when twister 105 is rotated as will be described further below. This may be accomplished by connecting the floor disc 114 and the twister 105 via interconnecting grooves and ribs, or letting the twister 105 extend longitudinally around the floor disc 114 as disclosed for example in Fig. 1. Preferably, the rotation of the dose disc 104 has two end positions corresponding to the dose administering position and the dose collecting position in its relation with the reservoir housing 103 in a known manner.

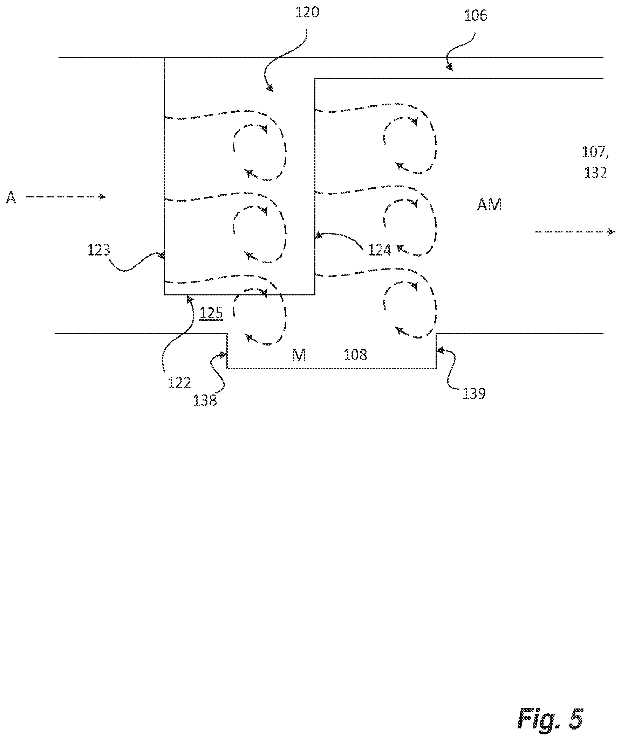

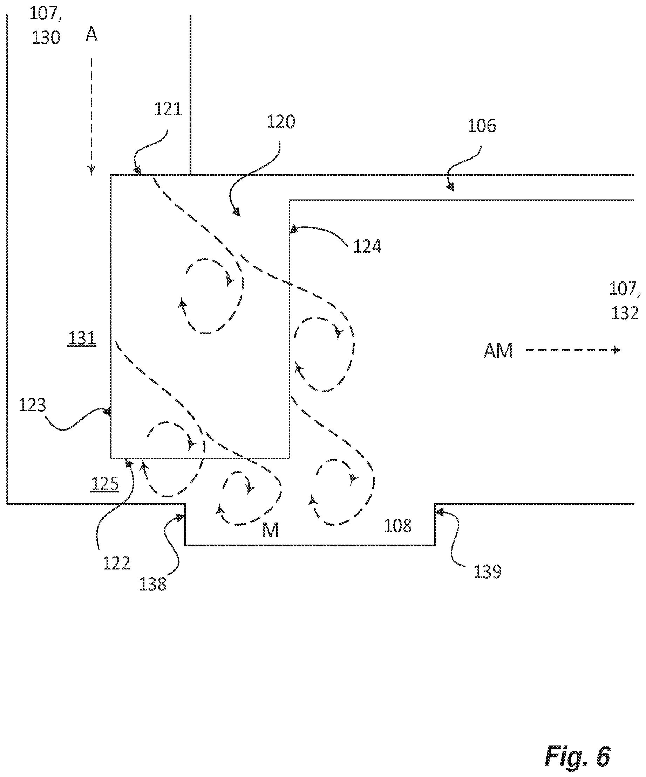

The dose administering position is illustrated in Fig. 1 and 2. In the dose administering position, the inlets 101 are in communication with the mixing and deaggregation chamber 106 via air channels 107. The air channels 107 direct the flow of air from inlets 101 initially downwards onto cavities 108 in the dose disc 104. Hence, in the dose administering position the cavities 108 may lie underneath and in line with the air channels 107, in particular in line with a longitudinal section 130 of the air channels 107. In addition, in the dose administering position the cavities 108 may be arranged flush with a transversal section 132 of a respective air channel 107 and may be arranged partially in line with a longitudinal section 130 of a respective air channel 107. The combination of an air flow A from channels 107 and the medicament M from cavities 108 then flows radially to the chamber 106 via the transversal section of the air channel 132 as will be described further below with respect to Fig. 5 and 6. When the dose disc 104 is rotated into a dose collecting position (not shown), the chamber 106 and the cavities 108 are rotated away from communication with the inlets 101 and air channels 107. Instead, the cavities 108 are rotated into medicament reservoir 109 and medicament reservoir 110 disclosed in Fig. 2, wherein the cavities 108 may collect a medicament housed in the reservoirs 109 and 110. The medicament contained in the medicament reservoir 109 may be a medicament different from the medicament contained in the medicament reservoir 110. Due to the presence of two reservoirs 109 and 110, the inhaler 100 may deliver two substances in one inhalation, the two substances otherwise being incompatible meaning that these two substances would not be possible to be comprised in one joint reservoir. Thus, the dry powder inhaler device 100 can effectively and satisfactorily disperse two dry powders and can administer a medicament comprising two or more substances which are incompatible in a mixture or are preferably stored in separate reservoirs for other reasons.

It is possible to arrange the dose disc 104 and the cavities 108 thereof such that when a first set of two cavities 108 lie underneath the air channels 107, i.e. in a dose administering position, a second set of two cavities 108 are positioned in the medicament reservoirs 109, 110 respectively. In this arrangement the inhaler has two medicament reservoirs, two air inlets, and one dose disc with four cavities. Additionally, the distribution of the cavities 108 on the dose disc 104 is such that the dose disc 104 may be rotated in one direction only meaning that when the second set of two cavities 108 lie underneath and in line with the air channels 107, the first set of cavities 108 are positioned in the medicament reservoirs 109, 110 respectively. It is also possible for the dose disc 104 to be rotated in a first direction so that cavities 108 lie underneath the air channels 107 in a dose administering position, and then for the dose disc 104 to be rotated in the opposite direction into the dose collecting position, and thereafter again for the dose disc to be rotated in the first direction back into the dose administering position. When the dose disc 104 is rotated in a first direction into the dose administering position and the opposite direction into the dose collecting position, the dose disc 104 may have rotational stops in the dose administering position and the dose collecting position, respectively, to ensure accurate alignment of the cavities 108 under air channels 107 and positioning in the medicament reservoirs 109, 110 respectively.

It is also envisioned that an inhaler provided with more than two, such as three, four, five, or six, reservoirs 109, 110 with the same arrangement of inlets, outlets, air channels, dose disc, cavities etc., is within the ambit of the present invention. For example, the inhaler 100 may have three medicament reservoirs 109, three air inlets 101, and a dose disc with three cavities 108. Alternatively, the inhaler 100 may have four medicament reservoirs 109, four air inlets 101, and a dose disc with four cavities 108. It is preferred however that the inhaler 100 have two air inlets 101, two air channels 107, one air outlet 102, two medicament reservoirs 109, 110, and one dose disc 104 with two cavities 108.

It is also envisioned that the inhaler 100 may be provided with a different dosage mechanism than the one disclosed above, for example electrical drive of different parts, and using paddles instead of the dose disc 104. However, use of the dosage mechanism 118 having the dose disc 104 and its cooperation with chimney 112 of the upper housing 103 and the reservoirs 109, 110 allows for a very cost effective solution while simultaneously ensuring high dose accuracy and the other benefits disclosed herein.

The air channels 107 have a first proximal conformation 134 as disclosed in the embodiment in Fig. 1. The proximal conformation 134 is such that the air channels 107 start at inlets 101 and extend downstream (during inhalation) in a central and transversal direction, where after they bend downwards at a right angle (90 degrees) to longitudinal sections 130 of the air channel 107 extending in a longitudinal and distal direction before connecting to transversal sections 132 of the air channel 107 via a second distal convention 131. In this way, when medicament lies in the cavities 108, the medicament is arranged flush with the transversal sections of the air channel 132 and the air flow direction will facilitate initial deaggregation of the medicament from the cavities 108. This facilitates that the medicament in the cavities 108 will be dispersed into the air flow and enters into the chamber 106.

Still referring to Fig. 1, the inhaler 100 according to the exemplary embodiments further comprises a turbulence enhancing protrusion (TEP) 120 disposed in the air channel 107 proximally above the cavity 108, in particular the TEP 120 may comprise a substantially rectangular bar disposed to extend from the transversal section 132 into the convention 131 and thus form a leading edge in respect of the downstream air flow in the longitudinal section 130. The TEP being configured to augment dissipation of medicament from cavity 108 in the dose administrating position by the facilitation of turbulence i.e. velocity fluctuations and disordered flow in layers of a fluid flow adjacent the cavity and at least partially inside the cavity. Thus, in one embodiment the number of TEPs 120 may equate to the number of cavities 108 so that a medicament cavity 108 is associated with a respective TEP 120 in the dose administrating position.

The geometrical shape and dimensions of the respective TEP 120 may be adapted in accordance with one or more parameters, for example the internal geometries of the inhaler 100 including the air channels 107, characteristics of the medicament to be dissipated or parameters relating to the user such as mass flow of air through the inhaler or air channel 107 caused by the user applying a pressure difference over the TEP as fluid is inhaled fluid from outlet 102 and fluid enters inlet 101.

The provision of a TEP 120 may have a throttling effect and causing a pressure drop in the air channels 107 aft the TEPs 120. However, benefits include of improved deaggregating effect and dissipation of medicament.

In fluid dynamics, an eddy is the swirling of a fluid and the reverse current created when the fluid is in a turbulent flow regime. Referring to Fig. 6, the moving fluid in the air channel 107 creates a space devoid of downstream-flowing fluid on a downstream side of the TEP 120 adjacent the cavity 108. Fluid behind or below the TEP 120 i.e. in proximity of the trailing edges, flows into the void creating a swirl of fluid on each edge of the TEP and/or the trailing edges, followed by a short reverse flow of fluid behind the TEP flowing upstream toward the back/trailing edges of the TEP 122, 124. Eddies are swirl regions which may transport mass, momentum and energy to other regions of flow whereas the so-called Eddy motion of particles refers to the motion of particles in layers of lower velocity to an adjacent layer of higher velocity.

The achieved turbulent flow regimes typically also provide increased shear stress in the fluid, in particular larger wall shear stress than in laminar flow regimes. Considering that the cavity 108 is arranged along the wall of the air channel 107, the larger shear stress may thus benefit the dissipation of medicament from the cavity 108.

The transition to a turbulent flow regime in a flow depends mainly to ratio between inertial forces to viscous forces in fluid. Thus, an object of the TEP 120 is to increase inertial forces in fluid e.g. by the formation of Eddie motions causing fluctuations in the values of velocity, temperature, pressure and density in the fluid.

These effects may advantageously be applied to enhance the dissipation of medicament from the cavity 108 by causing motion of medicament particles in a stationary or low velocity layer to an adjacent layer of higher velocity i.e. motion of a particle form the cavity 108 in a direction which may substantially transverse the general flow direction in the air channel 107. Thus, these effects aid in solving the problem of deaggregating and mixing medicament particles from a cavity 108 which borders to the air channel 107 in the dose administrating position wherein the cavity 108 is arranged underneath the air channel 107.

Referring to Fig.5, a simple implementation form of the invention may comprise the inhaler 100, having an air channel 107 extending between the air inlet 101 and the air outlet 102 with the cavity 108 arranged in the air channel 107, typically flush with the air channel 107 as disclosed in Fig 1, 2 and 5. The TEP 120 being arranged immediately upstream the cavity 108, such as to accomplish regions of turbulent flow regime in the region of the cavity 108, in particular immediately above and inside the cavity 108; pushing air of high velocity into the cavity 108.

Figure 5 and 6 shows an exemplary implementation form of the disclosure wherein the TEP 120 may advantageously be disposed in a suspended configuration in the air channel 107 to protrude into the air channel 107 from a locality opposing or partially opposing the cavity 108 so that the TEP 120 extends from an attached end to at least one free end, in a direction transverse the direction of the air channel 107 i.e. in a longitudinal direction of the inhaler 100, and towards the cavity 108. In one aspect, the TEP 120 or at least a part thereof extends directly towards the cavity 108 i.e. pointing at the cavity 108. Thereby, a fluid flow A, such as air in the air channel 107, may be divided upstream an anterior edge 138 of the cavity by means of at least one leading edge 121, 123 of the TEP 120. In addition, thereby, the divided fluid flow may again coincide aft the at least one trailing edge 122, 124 of the TEP 120 which may be adjacent the extent of the cavity 120, hence between the upstream anterior edge of the cavity 138 and the downstream posterior edge of the cavity 139. Accordingly, the TEP 120 may effectively disrupt the flow and cause local reverse flow above and in the cavity 108. Also, the TEP may effectively work as half open throttling valve, causing accelerations in the air flow about the TEP 120 i.a. due to the pressure gradient over the TEP 120 and thus increased fluid velocities and increased ratio of inertial forces to viscous forces in the fluid a the region of the cavity 108, in particular above and inside the cavity 108. Accordingly, the TEP 120 may effectively facilitate transition to a turbulent flow regime at least aft a trailing edge or edges 122, 124 of the TEP 120 where disordered flow and velocity fluctuations are prone to be generated.

The TEP 120 extends in a central and transversal direction of the inhaler 100 from a longitudinal leading edge of the TEP 123 to a longitudinal trailing edge of the TEP 124 in respect of the fluid flow A in the air channel 107 from the air inlet 101 to the air outlet 102 and in a central and transversal direction of inhaler 100.

In the dose administering position, the cavity 108 may extend downstream in respect of the longitudinal trailing edge of the TEP 124 as disclosed in Fig. 5.

In some embodiments the TEP 120 may extend at least to a centre axis or centre point of the air channel 107. The longitudinal leading edge 123 may have a length to height ratio ranging from [Q: Input needed in relation to dimensions] or corresponding to 2/3 - 3/4 of the diameter of the air channel 107.

The TEP 120 may comprise a rectangular or substantially rectangular bar-shape having one or more rounded corners and/or edges. The TEP 120 may have a thickness in the range of [Q: Input needed] The TEP 120 may be tapered in that the distance between the longitudinal edges 123, 124 may decrease in the direction into the air channel 107.

In the dose administering position, the longitudinal leading edge 123 may be arranged upstream in respect of the cavity 108. In the dose administering position, the cavity 108 may extend downstream in respect of the longitudinal trailing edge 124. In one exemplary embodiment the longitudinal trailing edge 124 is arranged between an upstream anterior edge of the cavity 138 and a posterior downstream edge of the cavity 139. In other exemplary embodiments the longitudinal leading edge 124 may be arranged upstream or in line with the anterior upstream edge of the cavity 138.

According to further aspects shown in Fig 6, the inhaler 100 may comprise one or more air channels 107 wherein each air channel 107 includes a longitudinal section 130 extending in a longitudinal and distal direction from the air inlet 101 via a first proximal conformation 134 and towards the cavity 108 where connecting to a transversal section 132 extending in a central and transversal direction via a distal conformation 131, which conformation 131 may be a right-angle conformation. The cavity 108 may be disposed in the further distal conformation 131 and may be aligned with the longitudinal section 130. The cavity 108 may be extending over a surface corresponding to the cross sectional area of the longitudinal section 130 or substantially corresponding to the cross sectional area of the longitudinal section 130. Alternatively, the cavity 108 may be disposed downstream the distal conformation 131 or partially downstream the distal conformation 131 so that the cavity 108 is partially in the distal conformation 131 and thus partially aligned with the longitudinal section 130 and partially extending into the transversal section 132 as disclosed in Fig. 5-7. The air channel 107 may extend from the distal second conformation 131 and radially inwards to a third central conformation 133 connecting to conduit 112 as shown in Fig.1.

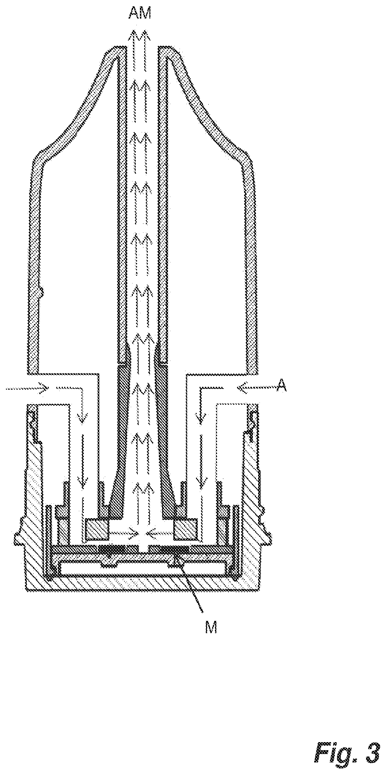

The TEP 120 may have substantially rectangular shape as disclosed in Fig. 1-12b, having one or more free ends or edges 122, 123. The TEP 120 may have the shape of a straight or flat bar having two parallel or substantially parallel main surfaces which may be a frontal side surface 140 and a dorsal side surface 141 respectively in relation to the direction of the flow A, and a thickness providing at least three edge-surfaces 122, 123, 124 or edges, in some embodiments four side surfaces or edges 121, 122, 123, 124. Other shapes of the TEP have been contemplated such airfoil shapes or bow-shapes. These edges 121, 122, 123, 124 may cause separation of the flow A and thus facilitate regions of reverse flow and turbulent flow regime as disclosed in Fig 5-7.

The TEP 120 may extend from its point of attachment in a transverse direction of the inhaler 100 and comprises an edge making a transverse leading edge 121 with respect of a flow A in the air channel 107, in particular with respect of the air flow A in the longitudinal section 130. Correspondingly, the TEP 120 may have a transverse trailing edge 122 typically extending in parallel or substantially in parallel with the transverse leading edge 121. The transverse leading edge is thus a proximal leading edge 121 and the transverse trailing edge a distal trailing edge 122 in relation to the proximal end 128 of the inhaler 100.

During use, air is typically expelled from an air outlet 102 at a proximal end 128 of the inhaler 100 through inhalation by a user whereby a pressure difference in the conduit 112 and air channels 107 causes air to enter the air inlets 101 and flow downstream into the longitudinal sections of the air channel 130 and to the adjoining transversal sections 132. According to aspects, the TEP 120 is disposed at least partially in the conformations 131 which constitutes conformations between the longitudinal sections 130 and the transversal sections 132 of the air channels 107. The TEP 120 may have a first free end protruding into the flow A flowing from the air inlet 101 and flowing downstream towards to the cavity 108 as disclosed in Fig. 1. Thus, the first free end or a portion thereof comprises a transverse leading edge in the form of a proximal leading edge 121 in respect of downstream flow in a longitudinal and distal direction of the inhaler 100. With the proximal leading edge 121 being arranged in the flow path between the air inlet 101 and the cavity 108 the opposite corresponding distal trailing edge 122 or portion thereof is also arranged in the flow A between the air inlet 101 and the cavity 108. However a portion of the proximal leading edge 121 may typically be used as means for suspending the TEP 120, i.e. a portion of the leading edge 121 may be attached to the inner wall of the air channel 107 and thus, the distal trailing edge 122 typically has a longer extent than does the proximal leading edge 121 in the embodiment disclosed in Fig. 6. The proximal leading edge 121 and the distal trailing edge 122 respectively may be parallel. The said first free end may further comprise a longitudinal edge in the form of a longitudinal leading edge 123. A second free end of the TEP may protrude towards the cavity 108 may comprise the transverse trailing edge 122. According to aspects, the TEP 120 is so dimensioned that a gap 125 is defined between the distal trailing edge 122 and the cavity 108. The gap 125 is thereby an intermediate gap between the distal trailing edge 122 and the dose disc 104 and/or the top medicament layer held in the cavity 108. The gap 125 may have a length in the range of [Q: approximate dimensions?] measured from the distal trailing edge 122 to the dose disc 104 adjacent the air channel 107 or the top medicament layer in the cavity 108.

The further two edges defined on the TEP 120 in relation to the flow A in the air channel 107; the longitudinal leading edge 123 with respect of the flow in the air channel 107, in particular with respect of the downstream flow in a central and transversal direction of the inhaler 100, i.e. in the transversal section 132; the longitudinal trailing edge 124 typically extending in parallel with the longitudinal leading edge 123 and connecting the proximal leading edge 121 to the distal trailing edge 122. The TEP 120 may however be tapered such that the distance between longitudinal edges 123, 124 decrease in a direction towards the cavity 108.

The proximal leading edge 121 may constitute a flat or square edge having a flat surface while the distal trailing edge 122, longitudinal leading edge 123 and longitudinal trailing edge 124 may typically feature rounded edges having curved surfaces.

The longitudinal leading edge 123 may be arranged upstream the corresponding longitudinal trailing edge 124. Typically, the longitudinal leading edge 123 is arranged in the distal convention 131 and makes a leading edge with respect to a fluid flow directed along the transversal section 132 and is disposed upstream the cavity 108. The longitudinal trailing edge 124 typically extending in parallel with the leading edge 123 and being disposed above the cavity 108, in particular in close vicinity of the anterior edge of the cavity 138. Generally, the fluid flow A in the air channel 107 will make a substantially diagonal flow path in respect of the substantially rectangular shaped TEP 120, hence diagonally over the two opposing main surfaces of the TEP 120; the frontal surface 140 facing upstream the flow in flow channel 107 and the dorsal surface 141 facing downstream the TEP in flow channel 107. However, a diagonal flow obviously comprises a respective longitudinal and transverse component which is accomplished by means of providing an air channel 107 having a longitudinal section 130 connecting to a transversal section 132. This arrangement will thus facilitate a flow A having a lateral component and a vertical component above the cavity 108 which causes increased fluid flow inside the cavity 108 being arranged underneath the air channel 107 in the dose administering position.

In a further possible implementation form of the invention, the air channel 107 comprises the longitudinal section 130 extending in a longitudinal and distal direction from the air inlet 101 to the transversal section of the air channel 132 via the distal conformation 131. The conformation 131 may be comprised of a portion of the longitudinal section 130 and a portion of the transversal section 132. The transversal section 132 may extending in a central and transversal direction of the inhaler along the dose disc 104 and passing the cavity 108.

The proximal leading edge of the TEP may protrude into the longitudinal section. A portion of the proximal leading edge 121 may be attached to the inhaler 100, in particular an inner wall of the air channel 107 or a medicament scraper 113. Thus, in a possible implementation form of the invention, the distal trailing edge 122 is longer than the proximal leading edge 121 and the longitudinal leading edge 123 is longer than the longitudinal trailing edge 124 as shown e.g. in Fig 5 and 6.

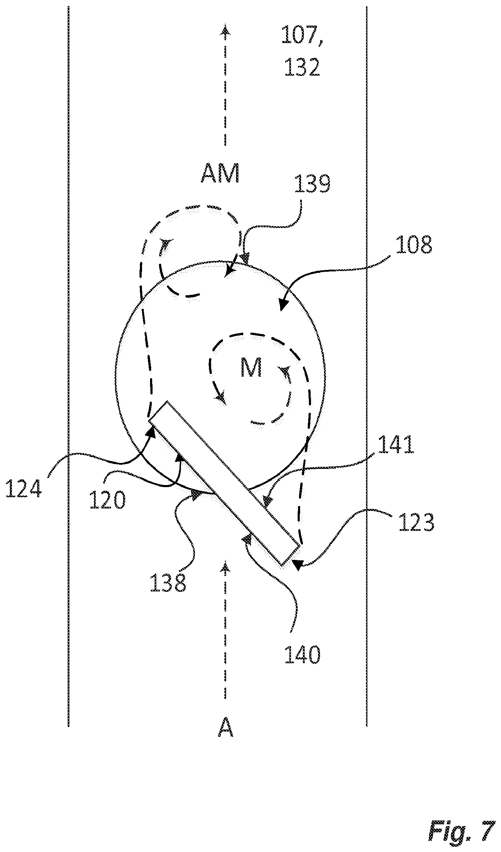

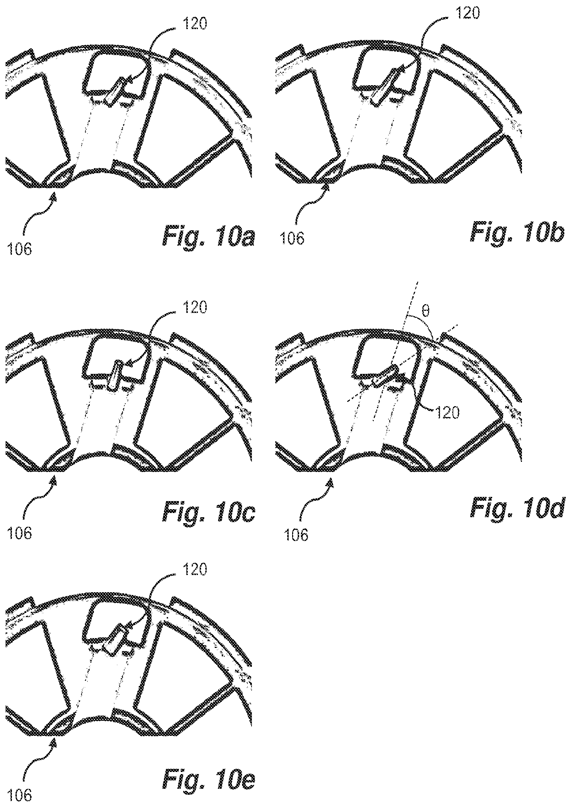

In the implementation forms shown in Fig. 7 and 10 also the longitudinal leading edge 123 is at least to some extend longer than the longitudinal trailing edge 124.

In Fig. 6 the cavity 108 is arranged in the dose administering position, whereby the distal trailing edge 122 may extend at least partially upstream the cavity 108, thus the free trailing edge 122 may at least partially oppose the cavity 108. Also, the longitudinal trailing edge 124 may be aligned above/over the cavity 108 in this position.

The TEP 120 may extend in a longitudinal and distal direction of the inhaler 100 from the proximal transverse leading edge of the TEP 121 to the distal transverse trailing edge of the TEP 122 in respect of a fluid flow in the air channel 107, in particular in respect of a fluid flow from the air inlet 101 through the longitudinal section 130 at least to the distal conformation 131.

A fluid flow, such as air flow A through the distal conformation 131 comprise a vertical component and a horizontal component, the fluid flow A in the beginning of the transversal section 132; in close proximity of the cavity 108, flowing about the TEP 120 and in the gap 125, may also comprise a vertical and/or distal component as disclosed in Fig. 6. Thus, the distal trailing edge 122 may constitute a trailing edge in respect of flow also in the transversal section 132.

In the dose administering position, the distal trailing edge 122 of the TEP 120 may be arranged in respect of the cavity 108 with the gap 125 there between, the gap 125 being in the range of 0.1 to 0.5 mm / uniform or substantially uniform along the extent of the gap 125.

Still referring to Fig. 7, the proximal leading edge 121 extends in a straight, or substantially straight transversal direction and constitute a transversally extending leading edge 121. Correspondingly, the distal trailing edge 122 may extend in a straight, or substantially straight transversal direction and may constitute an extending trailing edge 122, thereby forming leading- and trailing edges in respect of a flow direction in the longitudinal section 130 from an air inlet 101 to the distal conformation 131.

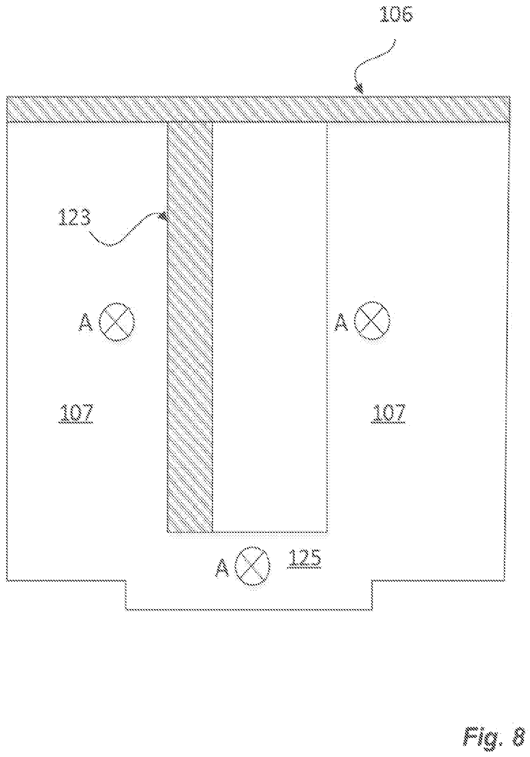

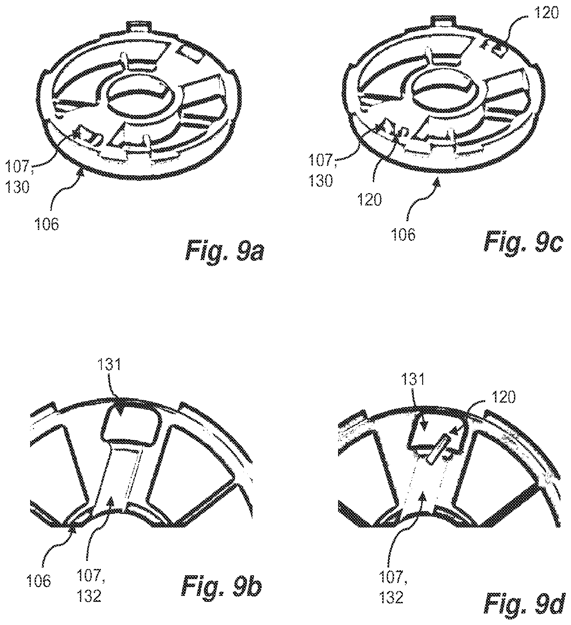

Figure 8 shows an implementation form of the invention wherein the TEP 120 is pivoted and angle θ in relation to the direction of the air channel 107 resulting in an angled positon also shown in Fig. 9 and 10. The TEP 120 is preferably fixedly attached in its position relative the air channel 107 and is thus not adjustable. The angled position of the TEP 120 may facilitate a throttling effect of the TEP 120 wherein the flow A is separated along the edges 123 and 124. The angled configuration further accomplishes that the flow A is separated over a greater width of the air channel 107 and along a greater extent of the air channel 107 and thus, turbulence is generated over a larger portion of the surface of the cavity 108.

Figures 9-11 shows various embodiments of the TEP 120 which may be formed as an integral part of the medicament scrapper 113. In Figures 9a-b it is illustrated a medicament scrapper absent a TEP and Figures 9c-d illustrates a medicament scrapper 113 featuring the TEP 120 disposed in the air channel 107, extending from transversal section 132 into the conformation 131 and/or the longitudinal section 130. As can be derived from Figure 10d, the TEP 120 may be configured pivoted in relation to the air channel 107 so as to make an angle θ in respect of the direction of the transversal section 132 of the air channel 107.

Figure 10a-e shows various embodiments of the TEP 120 having different dimensional properties. In Fig. 10a shows a configuration wherein the bar 120 has an angled configuration and extends approximately halfway into the longitudinal section 130 of the air channel 107, Fig. 10b shows a configuration wherein the bar 120 extends at least halfway into the longitudinal section 130 of the air channel 107, Fig. 10c shows a configuration wherein the bar 120 is not in an angled configuration and extends less than halfway into the longitudinal section 130 of the air channel 107, Fig. 10d shows a configuration wherein the bar 120 is configured with a large angle in respect of the direction of the transversal section 132 of the air channel 107 and Fig. 10e shows a configuration wherein the bar 120 has an increased width corresponding to approximately 25% of the width of the longitudinal section 130 of the air channel 107 and approximately 50% of the width of the transversal section 132 of the air channel 107. The skilled reader will appreciate that the invention is not limited to the embodiments of Fig. 10 which may be combined to yield yet further embodiment.

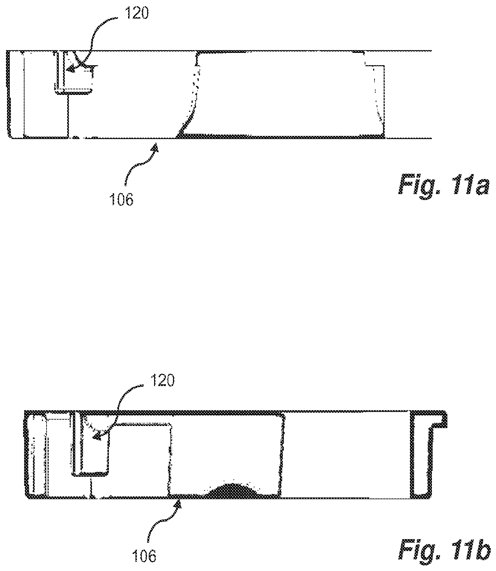

Figure 11a-b shows various exemplary embodiments of the TEP 120 having different depths. The TEP 120 in Fig. 11a features a depth corresponding approximately to half the height of the transversal section 132, in a longitudinal direction of the inhaler 100, or approximately half the height of the medicament scrapper 113. The TEP 120 in Fig. 11b features a depth corresponding approximately to 75% of the height of the transversal section 132, in a longitudinal direction of the inhaler 100, or approximately 75% of the height of the medicament scrapper 113.

The transverse trailing edge 122 may extend at least partially along the length of the cavity 108 and upstream the cavity 108 in the dose administering position or extend from the transversal section 132 and at least partially into the longitudinal section 130 alternatively extend exclusively into the longitudinal section 130 and/or the convention 131.

In the dose administrating position, the distal trailing edge of the TEP 122 may be arranged at least partially opposing the cavity 108. The TEP 120 may disposed at least partially in the distal convention 131 connecting the longitudinal section of the air channel 130 to the transversal section of the air channel 132. The distal trailing edge 122 may have a length corresponding to 1/3 - 1/2 of the diameter of the air channel 107, or being in the range of [Q: Any preferred dimensions?].

The TEP 120 may extend from an attached proximal end of the TEP to at least one free end, wherein the free end comprises three free edges 122, 123, 124 arranged substantially perpendicular in respect of each other.

The TEP 120 may extend from an attached proximal end to a first free end and to a second free end. The first free end and the second free end may be arranged substantially perpendicular. The first fee end may extend in a longitudinal direction and the second free end may extending in a transverse direction. Thus, the TEP 120 may comprise at four edges 121, 122, 123, 124 wherein all four edges 121, 122, 123, 124 may be arranged substantially perpendicular each other.

In a preferred embodiment, the cavity 108 extends further downstream in the transversal section of the air channel 132 than does the longitudinal trailing edge of the TEP 124. In a further preferred embodiment, the longitudinal leading edge 123 extends along the longitudinal section of the air channel 130 so that a gap 125 is defined between the transversal trailing edge 122 and the cavity 108 as has been explained above.

The skilled reader will appreciate that the disclosed invention is not limited to inhalers featuring both a longitudinal section 130 and a transversal section 132 but may be implemented in any inhaler to enhance dissipation of medicament from a cavity 108 arranged in an air channel 107 having an air inlet connecting to an air outlet 102.

In a further aspect, the TEP may configured to locally decrease a cross sectional area of the air channel 107, thereby a throttling effect is accomplished, thus increasing the velocity of a fluid flowing about and passing the TEP 120 whereby the increased velocity further enhances the dissipation of medicament from the cavity 108. This may be achieved i.a. by varying the dimensions of the TEP 120 e.g. by means of varying the width, length or depth respectively.

The TEP 120 may be centrally arranged in the cross section of the air channel 107 and/or above the cavity 108 and divert the flow in the air channel 107 into three flows; to the left hand side and to the right hand side and underneath the TEP in the interspace 125 as illustrated in Fig. 7 and 8.

In some aspects, the TEP 120 is pivoted about a longitudinal axis thereof, such as to make an angle of incidence θ with respect of the transverse section of the air channel 132 as illustrated in Fig. 7-10. Thus, with this particular configuration, the fluid, such as air, is prone to propagate according to the path of least resistance and thus causing formation of pressure and velocity gradients around the TEP 120 which efficiently cause a turbulent flow regime within a region thereof.

The TEP 120 may pivoted about a longitudinal axis thereof to form an angle θ in respect of the direction of the transversal section of the air channel 132 as disclosed in Fig 8. The angle θ may be in the range of 0 to 40 degrees. The angle θ may be variable for example to increase or decrease the respective velocities and pressures on each side of the TEP 120 so as to facilitate turbulence and formation or eddies at the desired location in the air channel 107. The angle θ may in other aspects be adapted in accordance with the properties of the medicament in a specific cavity 108. It has been contemplated that an inhaler 100 for example with two cavities each holding a respective medicament with different properties may also have TEPs 120 with tailored individual properties such as angle, length, depth and thickness.

Accordingly, the TEP 120 may effectively facilitate accelerations in the air flow about the TEP 120 and thus increased fluid velocities and increased ratio of inertial forces to viscous forces in the fluid above the cavity 108.

The entity providing the suspending of the TEP 120 may in some aspects include a medicament scrapper 113 as disclosed in Fig. 8-11b. According to aspects the TEP 120 constitutes an appendix extending from the medicament scrapper 113 and being formed as an integral part of the medicament scrapper 113. The medicament scrapper 113 may form an interproximal section of the air channel 107 or air channels associated with the cavities 108 and constituting at least part of a section of the air channel 107 being in close proximity of the cavity 108. However, other configurations are contemplatable.

The proximal leading edge 121, or a portion thereof, may be attached to a medicament scraper so that the TEP 120 is protrudes from the medicament scraper 113. In preferred embodiment, the TEP 120 forms an integral part or appendix of the medicament scrapper 113.

In addition, the present invention also address the problem of agglomeration of aggregated medicament commonly associated with prior art deaggregating deflectors. As have been mentioned, moisture and the cohesive forces of medicament particles typically exacerbate the agglomeration propensity of medicament on interior parts of inhalers, in particular on the deflectors. This problem is mitigated with respect of the TEP 120 according to the invention by means of arranging the TEP upstream or at least partially upstream in relation to the cavity 108. According to some embodiments, the TEP according to the invention does not feature a leading edge disposed downstream of the cavity 108 and thus medicament particles substantially will not propagate about the TEP but rather further downstream the TEP upon dissipation into the air channel 107.

A throttling effect of the TEP 120 may be achieved and the effect manipulated by varying the thickness of the TEP 120 and thus, the ratio between the cross sectional area of the air channel 107 and the TEP 120 arranged therein respectively may be varied for optimal effect.

Also, the longitudinal depth of the TEP i.e. the length with which it extends from its attached end into the air channel 107 may be optimized so that optimal deaggregating effect of the TEP 120 is achieved.

The cavity 108 may have circular shape or a substantially circular shape having a diameter substantially corresponding the diameter or the air channel 107, in particular the longitudinal section 130 and/or the transversal section 131.

This arrangement means for example that the reservoirs 109, 110 may comprise a dry powder medicament in the form of a micronized formulation or a carrier based formulation, or mixtures thereof. The inhaler 100 may then for example comprise a dry powder medicament in form of a micronized formulation in the first reservoir 109 and a free-flowing dry powder medicament in form of a carrier based formulation in the second reservoir 110.

It may not necessary for the air channels 107 to have a strictly right angle conformation as illustrated in Fig. 1. The air channels 107 could also curve inwards and downwards from inlets 101 before ending above and in line with the cavities 108. Other air channel conformations are considered within the ambit of the invention provided the inhaled air flow facilitates deaggregation of the medicament from the cavities 108.

Depending on the medicament to be administered, and the formulation thereof, the cavities 108 may take the form of a single circular shape when viewed from directly above or below the inhaler 100 as illustrated by the semi-circular shape of cavities 108 in Fig. 2. The single circular shape will be of approximately the same size and shape as the air channel 107. This will be most suitable when the medicament is not readily susceptible to aggregation and / or a large dose is desired. Other medicaments which tend to aggregate more may form an undesirable "plug" in the cavity 108 which is not readily dispersible during inhalation. Then it may be preferable to make several cavities 108 each having a relatively smaller diameter than a single circular shape as illustrated in Figs. 8. The several smaller cavities will continue to lie underneath one of the air channels 107 which remains unchanged in size and shape. An inhaler with several smaller cavities lying underneath one air channel 107 also allows for delivery of a smaller amount of powder. This feature also adds the possibility to combine or adapt the inhaler 100 for deliverance of micronized formulations and/or carrier based formulations.

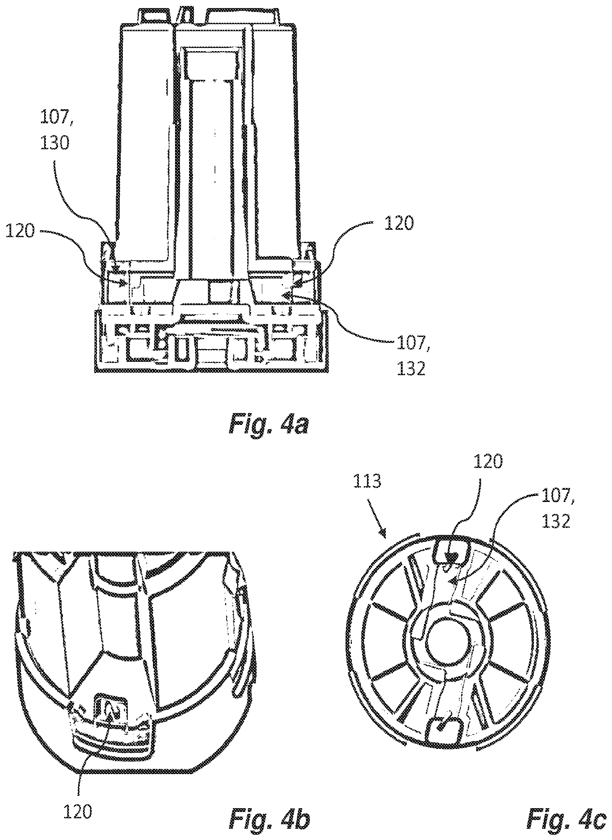

The reservoirs 109, 110 may be provided with medicament scrapers 113 illustrated in Figs. 4 and 5. The scrapers 113 are suspended at the bottom of the reservoirs 109, 110 such that they bear upon the dose disc 104. The scrapers 113 will pass over the cavities 108 of the dose disc 104 so that excessive medicament is removed from the cavities 108 to ensure correct dose volume. The scrapers 113 will also aid in compacting medicament in the cavities 108 which will improve retention of medicament in cavities 108 when the dose disc has been rotated into the dose administering position. Since the scrapers 113 are suspended at the bottom of the reservoirs 109, 110 they will automatically slide along the upper proximal surface of the dose disc 104, when the dose disc 104 is rotated between the dose administering position and dose collecting position. Preferably, each reservoir 109, 110 has a number of scrapers evenly distributed along the bottom of the reservoirs 109, 110. In this way the scrapers do not only aid in obtaining correct dose volume and dose compacting but also aid in distributing medicament at the bottom of the reservoirs 109, 110. The number of scrapers per reservoir 109, 110 could for example be selected in the interval of 1 to 6, such as 2 to 4, such as 3. It is also envisioned that the scrapers are arranged in an uneven distribution in the reservoirs 109, 110 if certain reservoirs are configured such that an uneven distribution of the scrapers will have a beneficial effect on the medicament distribution along the bottom of the reservoirs 109, 110.

During inhalation the medicament is emptied radially from the cavity 108 with the air flow from the air channels 107 into the chamber 106 wherein the air/medicament streams from the different air channels 107 and cavities 108 will cross, such that the medicament agglomerates will collide to increase deaggregation, where after a jet stream of finely dispersed medicament and air will continue through conduit 116 and inhalation chimney 112 out of the inhalator 100 through outlet 102 and into the lungs of the user. This radial emptying of the cavities 108 was described further above with respect to Fig. 5-8. The conduit 116 and the chimney 112 increases jet formation, allowing for a maintained low aggregation of medicament, hence increasing potential of medicament to reach far into the lungs of the patient. The conduit 116 and chimney 112 are generally tubular, but could optionally be provided with diverters, to further increase jet creation. Such diverters could be bumps or spiral-shaped ridges, extending along the length of the conduit and / or chimney from the chamber 106 to the air outlet 102. The chimney 112 does not necessarily have to be directed upwardly; it can just as well be directed downwardly or to the sides, whereby the outlet 102 is instead positioned at the bottom or on the sides, respectively. Additionally, the chimney 112 does not have to be generally tubular, but could be bent or sinus-shaped, depending on where on the inhaler 100 it is preferred to position the outlet 102. For flow characteristics and dose reliability and maintenance, it is however preferred to have it directed upwardly and generally tubular with optional diverters. The general shapes of the conduit 116 and the chimney 112 may also be such as to have differences in cross-sectional area, such as is present in a cone-shaped chimney. In this way, the flow velocity in the conduit and chimney may be regulated so as to help in deaggregation at chosen parts.

During use of the inhaler 100 the user will then simply rotate the upper housing 103 in one direction and thus the dose disc 104 into a dose collecting position if the dose disc is in a dose administering position. Thereafter, the upper housing 103 and the dose disc 104 are rotated preferably into the opposite direction to reach the dose administering position. If the dose disc 104 is already in the dose collecting position then of course the first rotation into the dose collecting position may be omitted. During these rotations, the scraper 113 will fill the cavities 108 of the dose disc 104 in the reservoirs 109, 110. After the dose disc 104 has been rotated into the dose administering position the cavities 108 are filled with medicament - optionally two different medicaments - and lie underneath the air channels 107. Then the user puts his/her mouth at outlet 102 and inhales. During inhalation air A will enter the inhaler 100 through inlets 101 and flow through air channels 107 to disperse and carry therewith the medicament(s) M from the cavities 108 in a radial direction in accordance with the arrows shown in Fig. 4, 6 and 7. The air/medicament flow AM will then enter the chamber 106. In the chamber 106, the air/medicament flows AM from the air channels 107 and cavities 108 will cross each other or coincide with each other, such that deaggregation of the medicaments M will increase which may increase dose uniformity since the need for diverters then is decreased. The flow characteristics, such as jet stream formation, will also increase. This feature allows the possibility to combine or adapt the inhaler 100 for deliverance of micronized formulations and/or carrier based formulations. Of course, it is also possible to combine the feature of crossing or coinciding flows from the two air channels 107 with diverters, even though the need thereof is decreased. Thereafter, the air/medicament flow AM - now comprising air/medicament flows from both air channels 107 and cavities 108, will go up through the conduit 116, the inhaler chimney 112, and finally through outlet 102 into the lungs of the user. A similar sequence of steps is then repeated the next time the inhaler 100 is required i.e. the user rotates the upper housing 103 and thus the dose disc 104 into a dose collecting position to fill the cavities 108 with a medicament(s) then the user rotates the dose disc 104 back into the dose administering position and inhales at outlet 102 as described immediately above.

The structure of, and functional relationship between, the cavities 108, reservoirs 109, 110 and the separate dose collecting and dose administering positions allows for no risk of multiple dosing by the user. In use the medicaments remain in the cavities 108 until inhalation. If inhalation is not commenced or is no longer required by the user, the cavities 108 carrying the medicaments may be rotated back into the reservoirs 109,110.

According to a further aspect, the invention relates to a method for dissipating medicament from a cavity (108). The cavity (108) may be disposed underneath an air channel (107) of a dry powder inhaler (100) and the air channel (107) connecting an air inlet (101) to an air outlet (102). The method may comprise providing a dry powder medicament in the cavity (108) and providing a turbulence enhancing protrusion (120) disposed to protrude into the air channel (107), upstream the cavity (108) preferably at least partially upstream of the cavity (108). During use, a pressure difference may be applied over the turbulence enhancing protrusion (120) e.g. by a user ingesting air from the inhaler (100), in particular from the outlet (102) thereby causing an air flow through the inlet (101) and through the air channel (107). As the caused air flow through the air channel (107) is incur with the one or more of the leading edges (121, 123) and/or trailing edges (122, 124), this will have the effect that the regime of the air flow will be disrupted, causing or enhancing turbulence aft the respective edge (121, 122, 123, 124)

The air flow in the air channel (107) may have a longitudinal direction and a transverse direction in relation the inhaler (100), thus a longitudinal component and a transverse component being parallel the extent of the cavity (108). The turbulence enhancing protrusion (120) man have a proximal leading edge (121) and a distal trailing edge (122) with respect of the longitudinal component and a leading edge (123) and a trailing edge (124) with respect of the transversal component. Typically, the longitudinal component is vertical or substantially vertical and the transverse component is horizontal or substantially horizontal during operation i.e. upon use of the inhaler.

Although, the present invention has been described above with reference to specific embodiments, it is not intended to be limited to the specific form set forth herein. Rather, the invention is limited only by the accompanying claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc. do not preclude a plurality. Reference signs in the claims are provided merely as a clarifying example and shall not be construed as limiting the scope of the claims in any way.

## Claims

1. A dry powder inhaler (100) comprising:
at least one air inlet (101) at a distal end zone of said inhaler (100), at least one air outlet (102) at a proximal end zone of said inhaler (100), and an air channel (107) between said at least one air inlet (101) and said at least one air outlet (102);
at least one medicament reservoir (109);
a dosage mechanism (118) for arranging at least one dose of a medicament from said at least one medicament reservoir (109) between the air channel (107) and the air outlet (102) such that said at least one dose may be delivered upon inhalation at said air outlet (102), wherein the dosage mechanism (118) comprises a dose disc (104) with at least one cavity (108), wherein the dose disc (104) may be rotated between a dose collecting position wherein the cavity (108) is positioned in the medicament reservoir (109), and a dose administering position wherein the cavity (108) is in communication with said air channel (107);
a turbulence enhancing protrusion (120) disposed suspended into said air channel (107), **characterized in that**
said turbulence enhancing protrusion (120) has a leading edge (123) and a trailing edge (124) in respect of a fluid flow (A) in the air channel (107) from the at least one air inlet (101) to the at least one air outlet (102),
wherein in said dose administrating position, said cavity (108) is extending downstream said trailing edge (124).

2. The inhaler (100) according to claim 1, wherein said leading edge (123) is arranged upstream said cavity (108).

3. The inhaler (100) according to claim 1 or 2, wherein said leading edge (123) and said trailing edge (124) are in respect of a transverse flow in said inhaler (100).

4. The inhaler (100) according to any one of the preceding claims, wherein said leading edge (123) is a longitudinal leading edge (123) and said trailing edge (124) is a longitudinal trailing edge (124) extending in a longitudinal direction of said inhaler (100).

5. The inhaler (100) according to any one of the preceding claims, said turbulence enhancing protrusion (120) further comprising a proximal leading edge (121) and a distal trailing edge (122) in respect of a longitudinal flow in said air channel (107).

6. The inhaler (100) according to claim 5, wherein said proximal leading edge (121) and/or said distal trailing edge (122) extend in a transversal direction of said inhaler (100).

7. The inhaler (100) according to any one of the preceding claims, said air channel (107) comprising:
a longitudinal section (130) extending from said at least one air inlet (101) in a longitudinal and distal direction of the inhaler (100);
a transversal section of said air channel (132) extending in a central and transversal direction of said inhaler (100) over said dose disc (104);
said longitudinal section (130) and said transversal section (132) connecting in a distal conformation (131);
wherein in said dose administrating position, the cavity (108) lies underneath the transversal section of said air channel (132).

8. The inhaler (100) according to any one of the preceding claims, wherein in said dose administrating positon, said trailing edge (124) is disposed between an upstream anterior edge of the cavity (138) and a downstream posterior edge of the cavity (139).

9. The inhaler (100) according to any one of the preceding claims, wherein in said dose administrating position, said turbulence enhancing protrusion (120) partially overlaps said cavity (108).

10. The inhaler (100) according to any one of the preceding claims, wherein said turbulence enhancing protrusion (120) is substantially rectangular, and said turbulence enhancing protrusion (120) extends from a fixedly attached proximal end of said turbulence enhancing protrusion (120) to at least one free end.

11. The inhaler (100) according to any one of the preceding claims, said turbulence enhancing protrusion (120) being adapted to locally decrease the cross section of said transversal sectional area of said air channel (132) by 10 to 30 %, preferably 15 to 25 %, more preferably 20 %.

12. The inhaler (100) according to any one of the preceding claims, wherein said turbulence enhancing protrusion (120) extends in a radial direction of said transversal section (132) into said air channel (107) from a side of the air channel (107) opposing said dose disc (104) and said cavity (108).

13. The inhaler (100) according to any one of the preceding claims, said turbulence enhancing protrusion (120) being adapted to provide regions of turbulent flow regimes in said flow channel (107) above and inside said cavity (108).

## Patentansprüche

1. Trockenpulverinhalator (100), umfassend:
mindestens einen Lufteinlass (101) an einem distalen Endbereich des Inhalators (100), mindestens einen Luftauslass (102) an einem proximalen Endbereich des Inhalators (100) und einen Luftkanal (107) zwischen dem mindestens einen Lufteinlass (101) und dem mindestens einen Luftauslass (102),
mindestens ein Medikamentenreservoir (109),
einen Dosierungsmechanismus (118) zum Anordnen mindestens einer Dosis eines Medikaments aus dem mindestens einen Medikamentenreservoir (109) zwischen dem Luftkanal (107) und dem Luftauslass (102), so dass die mindestens eine Dosis bei Inhalation an dem Luftauslass (102) abgegeben werden kann, wobei der Dosierungsmechanismus (118) eine Dosierscheibe (104) mit mindestens einem Hohlraum (108) umfasst, wobei die Dosierscheibe (104) zwischen einer Dosissammelposition, in der der Hohlraum (108) in dem Medikamentenreservoir (109) positioniert ist, und einer Dosisabgabeposition, in der der Hohlraum (108) mit dem Luftkanal (107) in Verbindung steht, gedreht werden kann,
einen turbulenzverstärkenden Vorsprung (120), der in dem Luftkanal (107) eingesetzt ist, **dadurch gekennzeichnet, dass**
der turbulenzverstärkende Vorsprung (120) eine Vorderkante (123) und eine Hinterkante (124) in Bezug auf eine Fluidströmung (A) in dem Luftkanal (107) von dem mindestens einen Lufteinlass (101) zu dem mindestens einen Luftauslass (102) aufweist,
wobei sich in der Dosisabgabeposition der Hohlraum (108) stromabwärts der Hinterkante (124) erstreckt.

2. Inhalator (100) nach Anspruch 1, wobei die Vorderkante (123) stromaufwärts des Hohlraums (108) angeordnet ist.

3. Inhalator (100) nach Anspruch 1 oder 2, wobei die Vorderkante (123) und die Hinterkante (124) in Bezug auf eine Querströmung in dem Inhalator (100) angeordnet sind.

4. Inhalator (100) nach einem der vorstehenden Ansprüche, wobei die Vorderkante (123) eine Längsvorderkante (123) und die Hinterkante (124) eine Längshinterkante (124) ist, die sich in einer Längsrichtung des Inhalators (100) erstrecken.

5. Inhalator (100) nach einem der vorstehenden Ansprüche, wobei der turbulenzverstärkende Vorsprung (120) ferner eine proximale Vorderkante (121) und eine distale Hinterkante (122) in Bezug auf eine Längsströmung in dem Luftkanal (107) umfasst.

6. Inhalator (100) nach Anspruch 5, wobei sich die proximale Vorderkante (121) und/oder die distale Hinterkante (122) in einer Querrichtung des Inhalators (100) erstrecken.

7. Inhalator (100) nach einem der vorstehenden Ansprüche, wobei der Luftkanal (107) umfasst:
einen Längsabschnitt (130), der sich von dem mindestens einen Lufteinlass (101) in einer Längs- und Distalrichtung des Inhalators (100) erstreckt,
einen Querabschnitt des Luftkanals (132), der sich in einer mittleren und Querrichtung des Inhalators (100) über die Dosierscheibe (104) erstreckt,
wobei der Längsabschnitt (130) und der Querabschnitt (132) in einer distalen Konformation (131) miteinander verbunden sind,
wobei in der Dosisabgabeposition der Hohlraum (108) unterhalb des Querabschnitts des Luftkanals (132) liegt.

8. Inhalator (100) nach einem der vorstehenden Ansprüche, wobei in der Dosisabgabeposition die Hinterkante (124) zwischen einer stromaufwärtigen Vorderkante des Hohlraums (138) und einer stromabwärtigen Hinterkante des Hohlraums (139) angeordnet ist.

9. Inhalator (100) nach einem der vorstehenden Ansprüche, wobei in der Dosisabgabeposition der turbulenzverstärkende Vorsprung (120) den Hohlraum (108) teilweise überlappt.

10. Inhalator (100) nach einem der vorstehenden Ansprüche, wobei der turbulenzverstärkende Vorsprung (120) im Wesentlichen rechteckig ist und sich von einem fest angebrachten proximalen Ende des turbulenzverstärkenden Vorsprungs (120) zu mindestens einem freien Ende erstreckt.

11. Inhalator (100) nach einem der vorstehenden Ansprüche, wobei der turbulenzverstärkende Vorsprung (120) dazu ausgelegt ist, den Querschnitt des Querschnittsbereichs des Luftkanals (132) lokal um 10 bis 30 %, vorzugsweise 15 bis 25 %, noch bevorzugter 20 % zu verringern.

12. Inhalator (100) nach einem der vorstehenden Ansprüche, wobei sich der turbulenzverstärkende Vorsprung (120) in einer radialen Richtung des Querschnitts (132) in den Luftkanal (107) von einer Seite des Luftkanals (107) aus erstreckt, die der Dosierscheibe (104) und dem Hohlraum (108) gegenüberliegt.

13. Inhalator (100) nach einem der vorstehenden Ansprüche, wobei der turbulenzverstärkende Vorsprung (120) dazu ausgelegt ist, Bereiche mit turbulenten Strömungsregimen in dem Strömungskanal (107) oberhalb und innerhalb des Hohlraums (108) bereitzustellen.

## Revendications

1. Inhalateur de poudre sèche (100) comprenant :
au moins une entrée d'air (101) au niveau d'une zone d'extrémité distale dudit inhalateur (100), au moins une sortie d'air (102) au niveau d'une zone d'extrémité proximale dudit inhalateur (100), et un canal d'air (107) entre ladite au moins une entrée d'air (101) et ladite au moins une sortie d'air (102) ;
au moins un réservoir de médicament (109) ;
un mécanisme de dosage (118) pour agencer au moins une dose d'un médicament à partir dudit au moins un réservoir de médicament (109) entre le canal d'air (107) et la sortie d'air (102) de sorte que ladite au moins une dose puisse être délivrée lors de l'inhalation au niveau de ladite sortie d'air (102), dans lequel le mécanisme de dosage (118) comprend un disque doseur (104) avec au moins une cavité (108), dans lequel le disque doseur (104) peut être tourné entre une position de collecte de dose dans laquelle la cavité (108) est positionnée dans le réservoir de médicament (109), et une position d'administration de dose dans laquelle la cavité (108) est en communication avec ledit canal d'air (107) ;
une saillie améliorant les turbulences (120) disposée suspendue dans ledit canal d'air (107), **caractérisée en ce que**
ladite saillie améliorant les turbulences (120) présente un bord d'attaque (123) et un bord de fuite (124) par rapport à un écoulement de fluide (A) dans le canal d'air (107) de l'au moins une entrée d'air (101) à l'au moins une sortie d'air (102),
dans lequel dans ladite position d'administration de dose, ladite cavité (108) s'étend en aval dudit bord de fuite (124).

2. Inhalateur (100) selon la revendication 1, dans lequel ledit bord d'attaque (123) est agencé en amont de ladite cavité (108).

3. Inhalateur (100) selon la revendication 1 ou 2, dans lequel ledit bord d'attaque (123) et ledit bord de fuite (124) sont par rapport à un écoulement transversal dans ledit inhalateur (100).

4. Inhalateur (100) selon l'une quelconque des revendications précédentes, dans lequel ledit bord d'attaque (123) est un bord d'attaque longitudinal (123) et ledit bord de fuite (124) est un bord de fuite longitudinal (124) s'étendant dans une direction longitudinale dudit inhalateur (100).

5. Inhalateur (100) selon l'une quelconque des revendications précédentes, ladite saillie améliorant les turbulences (120) comprenant en outre un bord d'attaque proximal (121) et un bord de fuite distal (122) par rapport à un écoulement longitudinal dans ledit canal d'air (107).

6. Inhalateur (100) selon la revendication 5, dans lequel ledit bord d'attaque proximal (121) et/ou ledit bord de fuite distal (122) s'étendent dans une direction transversale dudit inhalateur (100).

7. Inhalateur (100) selon l'une quelconque des revendications précédentes, ledit canal d'air (107) comprenant :
une section longitudinale (130) s'étendant à partir de ladite au moins une entrée d'air (101) dans une direction longitudinale et distale de l'inhalateur (100) ;
une section transversale dudit canal d'air (132) s'étendant dans une direction centrale et transversale dudit inhalateur (100) sur ledit disque doseur (104) ;
ladite section longitudinale (130) et ladite section transversale (132) se raccordant dans une conformation distale (131) ;
dans lequel dans ladite position d'administration de dose, la cavité (108) se trouve sous la section transversale dudit canal d'air (132).

8. Inhalateur (100) selon l'une quelconque des revendications précédentes, dans lequel dans ladite position d'administration de dose, ledit bord de fuite (124) est disposé entre un bord antérieur amont de la cavité (138) et un bord postérieur aval de la cavité (139).

9. Inhalateur (100) selon l'une quelconque des revendications précédentes, dans lequel, dans ladite position d'administration de dose, ladite saillie améliorant les turbulences (120) chevauche partiellement ladite cavité (108).

10. Inhalateur (100) selon l'une quelconque des revendications précédentes, dans lequel ladite saillie améliorant les turbulences (120) est sensiblement rectangulaire, et ladite saillie améliorant les turbulences (120) s'étend d'une extrémité proximale fixement attachée de ladite saillie améliorant les turbulences (120) à au moins une extrémité libre.

11. Inhalateur (100) selon l'une quelconque des revendications précédentes, ladite saillie améliorant les turbulences (120) étant adaptée pour diminuer localement la section transversale de ladite zone transversale dudit canal d'air (132) de 10 à 30 %, de préférence de 15 à 25 %, de préférence encore de 20 %.

12. Inhalateur (100) selon l'une quelconque des revendications précédentes, dans lequel ladite saillie améliorant les turbulences (120) s'étend dans une direction radiale de ladite section transversale (132) dans ledit canal d'air (107) à partir d'un côté du canal d'air (107) opposé audit disque doseur (104) et à ladite cavité (108).

13. Inhalateur (100) selon l'une quelconque des revendications précédentes, ladite saillie améliorant les turbulences (120) étant adaptée pour fournir des régions de régimes d'écoulement turbulent dans ledit canal d'écoulement (107) au-dessus et à l'intérieur de ladite cavité (108).
